# EUROPEAN PATENT SPECIFICATION

(11) **EP 1 175 380 B1**
(45) Date of publication and mention of the grant of the patent: **10.12.2003**
(21) Application number: 00954114.5
(22) Date of filing: 17.08.2000
(51) Int. Cl.: C07C 17/23, C07C 21/18, C07C 22/08, C07C 41/24, C07C 43/17

(54) **PREPARATION OF SELECTED FLUOROOLEFINS**
HERSTELLUNG VON AUSGEWÄHLTEN FLUOROLEFINEN
PREPARATION DE FLUOROOLEFINES SELECTIONNEES

(30) Priority: 20.08.1999 RU 99118168
(43) Date of publication of application: 30.01.2002
(73) Proprietor: E.I. DU PONT DE NEMOURS AND COMPANY, Wilmington Delaware 19898 (US)
(72) Inventor: BELEN'KII, Gennadii Gerskovich, Moscow, 117192 (RU); PETROV, Viacheslav Alexandrovich, Hockessin, DE 19707 (US); POSTOVOI, Sergei Arnol'dovich, Moscow, 121536 (RU); RESNICK, Paul, Raphael, Cary, NC 27511 (US); ZEIFMAN, Yurii Vilovich, Apartment 3, Moscow, 117571 (RU)
(74) Representative: Carpmaels & Ransford
(86) International application number: US0022530
(87) International publication number: WO01014294

(56) References cited:
- EP-A- 0 434 407
- US-A- 3 192 274
- US-A- 4 820 884
- US-A- 5 536 885
- MIDDLETON, WILLIAM J. ET AL: "1-Trifluoromethyl-1,2,2-triphenylethylene s. Synthesis and postcoital antifertility activity" J. MED. CHEM. (1971), 14(12), 1193-7 , XP002156406

## Description

### FIELD OF THE INVENTION

This invention concerns a process for the manufacture of certain fluorocarbons which have an olefinic bond by defluorination of a fluorocarbon starting material having a corresponding saturated bond.

### BACKGROUND

Fluorinated olefins are used to prepare fluoropolymers. These polymers typically have a unique combination of properties. These include inter alia high thermal stability, chemical inertness, unusual surface properties, low dielectric constant and low flammability.

2-Trifluoromethyl-3,3,3-trifluoropropene (i.e., (CF₃)₂C=CH₂ or HFIB) is an example of a fluoroolefin that has been used to prepare various polymers. For example, it has been copolymerized with vinylidene fluoride to produce an alternating copolymer having exceptional thermal , chemical and mechanical properties. Numerous routes to HFIB have been disclosed. For example, U.S. Patent No. 4,705,904 discloses the manufacture of HFIB by reacting hexafluoroacetone or hexafluoropropylene oxide, its precursor, with ketene or a ketene precursor at 500°C to 700°C. Traces of perfluoroisobutylene, a highly toxic olefin, are found in the crude product mixture. U.S. Patent No. 4,766,238 discloses a process for the preparation of HFIB by the reaction of monochloromethyl 2-trifluoromethyl-3,3,3-trifluoropropionate with an amine. The propionate ester is prepared by chlorination of the methyl ester. The use of chlorine or chlorine-containing precursors presents a waste disposal problem. Further disadvantages of the art processes are described in U.S. Patent No. 4,705,904.

U.S. Patent No. 4,820,884 discloses a process for preparing an unsaturated aliphatic or cycloaliphatic perfluorocarbon having at least six carbon atoms and having at least one carbon-carbon double bond comprising contacting the corresponding perfluoroalkane or perfluorocycloalkane having at least two adjacent tertiary carbon atoms with activated carbon at a temperature of from about 300°C to about 500°C.

There is an interest in developing more efficient processes for the manufacture of unsaturated fluorocarbons, particularly, fluorinated olefins. Furthermore, the unsaturated fluorocarbons prepared by the process of this invention also contain hydrogen. These compounds, unlike perfluorinated unsaturated fluorocarbons, pose less of an environmental problem and are typically less expensive than their perfluorinated analogues.

### SUMMARY OF THE INVENTION

A process is provided for producing an olefinic fluorocarbon selected from the group consisting of (CF₃)₂C=CH₂, CF₃CH=CF₂, CF₂=C(CF₃)OCF₂CHF₂ and C₆H₅C(CF₃)=CF₂. The process comprises contacting the corresponding fluorocarbon starting material selected from the group consisting of (CF₃)₂CFCH₂F, (CF₃)₂CHF, (CF₃)₂CFOCF₂CHF₂ and C₆H₅CF(CF₃)₂, in the vapor phase, with a defluorination reagent selected from the group consisting of carbon, copper, iron, nickel and zinc at an elevated temperature of at least 300°C.

### DETAILED DESCRIPTION

In accordance with the process of this invention, certain hydrogen-containing fluorocarbon starting materials surprisingly defluorinate (i.e., lose two fluorine atoms) rather than dehydrofluorinate (i.e., lose hydrogen fluoride). In particular, it has been found that (CF₃)₂C=CH₂ can be produced from (CF₃)₂CFCH₂F; CF₃CH=CF₂ can be produced from (CF₃)₂CHF; CF₂=C(CF₃)OCF₂CHF₂ can be produced from (CF₃)₂CFOCF₂CHF₂; and 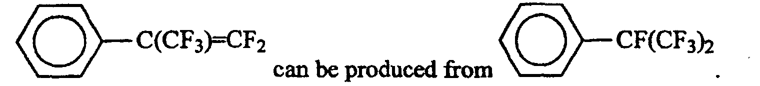 (CF₃)₂CFCH₂F can be prepared by the reaction of difluoromethane, antimony pentafluoride and perfluoropropylene in an autoclave at 80°C. Further details are described in International Publication No. WO 98/42645. (CF₃)₂CHF (227ea) can be prepared by the addition of HF to hexafluoropropene in the presence of activated carbon as described in British Patent Specification No. 902,590. (CF₃)₂CFOCF₂CHF₂ is a known compound (see Example 7). C₆H₅CF(CF₃)₂ can be prepared by a procedure described in W. A. Sheppard, J. Am. Chem. Soc., 87, 2410 (1965).

The carbon defluorination reagent includes activated carbon and acid-washed carbons (e.g., carbons which have been treated with hydrochloric acid or hydrochloric acid followed by hydrofluoric acid). Suitable acid treatment of carbons is described in U.S. Patent No. 5,136,113. The carbon catalyst also includes three dimensional matrix porous carbonaceous materials. Examples are those described in U.S. Patent No. 4,978,649. Of note are three dimensional matrix carbonaceous materials which are obtained by introducing gaseous or vaporous carbon-containing compounds (e.g., hydrocarbons) into a mass of granules of a carbonaceous material (e.g., carbon black); decomposing the carbon-containing compounds to deposit carbon on the surface of the granules; and treating the resulting material with an activator gas comprising steam to provide a porous carbonaceous material. A carbon-carbon composite material is thus formed.

The carbon, copper, iron, nickel and zinc defluorination reagents can be in any convenient form such as powder, granules and chips. The iron and nickel reagents may also be in gauze form. The metal defluorination reagents (i.e., Cu, Fe, Ni and Zn) can be regenerated by reaction with hydrogen at temperatures of 300°C to 600°C.

In the process of the invention a fluorocarbon starting material selected from (CF₃)₂CFCH₂F, (CF₃)₂CHF, (CF₃)₂CFOCF₂CHF₂ and C₆H₅CF(CF₃)₂ is contacted with the defluorination reagent at a temperature of from 300°C to 800°C, preferably from 350°C to 500°C. The fluorocarbon starting matenai is reacted in me vapur phase and may be contacted with the defluorination reagent neat or diluted with an inert gas such as argon and nitrogen.

The contact time is typically from 0.1 seconds to 30 minutes.

The vapor phase process of this invention can be carried out using well-known chemical engineering practice. The process is conveniently done at atmospheric pressure, although subatmospheric or superatmospheric pressures can be employed. Reactor vessels of stainless steel are typically used although other materials such as nickel-based corrosion resistant alloys such as Hastelloy™ nickel alloy can be used.

Without further elaboration, it is believed that one skilled in the art can, using the description herein, utilize the present invention to its fullest extent. The following specific embodiments are to be construed as illustrative, and not as constraining the remainder of the disclosure in any way whatsoever.

### EXAMPLES

### Legend

| | |
|---|---|
| 338myq is (CF₃)₂CFCH₂F | HFIB is (CF₃)₂C=CH₂ |
| 227ea is (CF₃)₂CHF | 1225zc is CF₃CH=CF₂ |

The reactor used for all the examples was a stainless steel tube 510 mm long x 14 mm diameter; the operating zone was 360 mm.

### EXAMPLE 1

### (CF₃)₂CFCH₂F → (CF₃)₂C=CH₂

Iron filings (70 g) were placed in the steel tube. Hydrogen was passed over the iron filings for 3 hours at 500°C. The hydrogen flow was stopped and (CF₃)₂CFCH₂F (338myq, 5 g) in argon was passed through the tube for 2.5 hours. The reactor products were condensed in a trap (-78°C) and a mixture (4.5 g) containing (CF₃)₂C=CH₂ (hexafluoro-isobutene or HFIB, 22%) and (CF₃)₂CFCH₂F (78%) as determined by gas chromatography and ¹⁹F NMR was found. The yield of HFIB was 67% and the conversion of 338myq was 30%.

### EXAMPLE 2

### (CF₃)₂CFCH₂F → (CF₃)₂C=CH₂

Activated carbon (22 g) was placed in the steel tube. 338myq (5 g) in argon was passed through the tube at 350°C for 5 hours. The reactor products were condensed in a trap (-78°C) and a mixture (4.5 g) containing HFIB (34%) and 338myq (66%) as determined by gas chromatography, mass spectroscopy, ¹H NMR and ¹⁹F NMR was found. The yield of HFIB was 57%.

The reaction was repeated at 450°C. The reactor products contained HFIB (87%) and 338myq (13%) as determined by gas chromatography, mass spectroscopy, ¹H NMR and ¹⁹F NMR. The yield of HFIB was 67%.

### EXAMPLE 3

### (CF₃)₂CFCH₂F → (CF₃)₂C=CH₂

Nickel chips (37 g) were placed in the steel tube. 338myq (6.2 g) in argon was passed through the tube at 500°C for 2 hours. The reactor products were condensed in a trap (-78°C) and a mixture (5.8 g) containing HFIB (6.3%) and 338myq (93.7%) was found. The yield of HFIB was 57% and the 338myq conversion was 13%.

### EXAMPLE 4

### (CF₃)₂CFCH₂F → (CF₃)₂C=CH₂

Zinc chips (72 g) were placed in the steel tube. 338myq (7.5 g) in argon was passed through the tube at 500°C for 2 hours. The reactor products were condensed in a trap (-78°C) and a mixture (6 g) containing HFIB (12%) and 338myq (88%) was found. The yield of HFIB was 72% and the 338myq conversion was 16%.

### EXAMPLE 5

### (CF₃)₂CFCH₂F → (CF₃)₂C=CH₂

Copper chips (54 g) were placed in the steel tube. 338myq (5 g) in argon was passed through the tube at 500°C for 2 hours. The reactor products were condensed in a trap (-78°C) and a mixture (4.5 g) containing HFIB (10%) and 338myq (90%) was found. The yield of HFIB was 58% and the 338myq conversion was 15.4%.

### EXAMPLE 6

### (CF₃)₂CHF → CF₃CH=CF₂

Iron filings (80 g) were placed in the steel tube. 227ea (10 g) was passed through the tube at 500°C for 4 hours. The reactor products were condensed in a trap (-78°C) and a mixture (6.1 g) containing 1225zc (9.5%) and 227ea (90.5%) was found. The yield of 1225zc was 16% and the 227ea conversion was 55%.

### COMPARATIVE EXAMPLE A

### Preparation of CF₃CF₂CH₂F

CF₃CF₂CH₂F can be prepared by the reaction of difluoromethane, antimony pentafluoride and tetrafluoroethylene in an autoclave at 50°C. Further details are described in International Publication No. WO 98/42645.

CF₃CF₂CH₂F did not react when contacted with either iron filings or carbon under the same conditions as described in Example 6.

### EXAMPLE 7

### (CF₃)₂CFOCF₂CHF₂ → CF₂=C(CF₃)OCF₂CHF₂

### Preparation of (CF₃)₂CFOCF₂CHF₂

A solution of (CF₃)₂CFOCF₂CF₂I (21.3 g) in isopropanol (8 mL) was added dropwise to a suspension of Al chips (1 g) and HgCl₂ (0.4 g) in isopropanol (30 mL). After the exothermic reaction was completed, the reaction mixture was stirred for 1 hour at 55°C. The reaction mixture was then distilled and a fraction with a b.p. of less then 80°C was collected. This fraction was washed with an aqueous HCl solution and then distilled over cone. H₂SO₄ to give (CF₃)₂CFOCF₂CHF₂ (11.8 g, 80% yield), b.p. 44 to 46°C. The ¹H and ¹⁹F NMR were in accord with the assigned structure.

Iron chips (70 g) were placed in the steel tube and activated with hydrogen at 500°C. (CF₃)₂CFOCF₂CHF₂ (4.6 g) in argon was passed through the tube at 500°. The reactor products were condensed in a trap (-78°C) and a mixture (3.9 g) containing (CF₃)₂CFOCF₂CHF₂ (96.7%) and CF₂=C(CF₃)OCF₂CHF₂ (3.3%) was found. The yield of CF₂=C(CF₃)OCF₂CHF₂ was 18%.

### COMPARATIVE EXAMPLE B

C₃F₇OCHFCF₃ did not react when contacted with either iron filings or carbon under the same conditions as described in Example 6.

### COMPARATIVE EXAMPLE C

### (CF₃)₂CFOC₂H₅ → (CF₃)₂C=O + C₂H₅F

(CF₃)₂CFOC₂H₅ was prepared by the method described in French Patent Publication No. 1,508,638 (1967); Chem. Abstr. 70:1122a.

(CF₃)₂CFOC₂H₅ did not defluorinate when contacted with iron filings under the same conditions as described in Example 6. The ether decomposed to hexafluoroacetone and ethyl fluoride even at temperatures as low as 200°C.

### COMPARATIVE EXAMPLE D

### (CF₃)₂CFCH₂CH₃ → (CF₃)₂C=CH₂ + CF₂=C(CF₃)C₂H₅ + (CF₃)₂C=CHCH₃ + (CF₃)₂CHC₂H₅

### Preparation of (CF₃)₂CFCH₂CH₃

A mixture of perfluoroisopropyl iodide (56 g, 0.19 mole), ethylene (5 L, 0.2 mole) and benzoyl peroxide (3.2 g) was heated in a 100 mL steel autoclave at 100°C for 6 hours with stirring. The reaction product contained perfluoroisopropyl iodide and (CF₃)₂CFCH₂CH₂I in a 1:1 ratio. Distillation of the reaction mixture gave (CF₃)₂CFCH₂CH₂I (b.p. 118 to 124°C) in 33% yield. (CF₃)₂CFCH₂CH₂I (10 g) was added dropwise to a mixture of zinc dust (5 g) and acetic acid (30 mL). After the exothermic reaction was completed, the reaction mixture was stirred for 2.5 hours at 60°C, diluted with water and distilled to collect a fraction with a b.p. less than 100°C. The distillate was separated from water, dried over calcium chloride, and redistilled to give (CF₃)₂CFCH₂CH₃ (b.p. 39 to 42°C, 56% yield). The ¹H and ¹⁹F NMR were in accord with the assigned structure.

Iron chips (70 g) were placed in the steel tube and activated with hydrogen at 500°C. (CF₃)₂CFCH₂CH₃ (3.8 g) in argon was passed through the tube at 500°. The reactor products were condensed in a trap (-78°C) and a mixture (1.5 g) containing (CF₃)₂CFCH₂CH₃ (75%) and (CF₃)₂C=CH₂ (25%) was found. The yield of (CF₃)₂C=CH₂ was 17.3%. Traces of CF₂=C(CF₃)C₂H₅, (CF₃)₂C=CHCH₃ and (CF₃)₂CHC₂H₅ were also found.

### EXAMPLE 8

### C₆H₅CF(CF₃)₂ → C₆H₅C(CF₃)=CF₂ + C₆H₅CH(CF₃)₂

Iron chips (60 g) were placed in the steel tube, heated in argon for 4 hours and then activated with hydrogen at 500°C for 1.5 hours. C₆H₅CF(CF₃)₂ (3.6 g) in argon was passed through the tube at 500°. The reactor products were condensed in a trap (-78°C) and a mixture (1.5 g) containing C₆H₅CF(CF₃)₂ (79%), C₆H₅C(CF₃)=CF₂ (14%) and C₆H₅CH(CF₃)₂ (7%) was found. The yield of C₆H₅C(CF₃)=CF₂ was 11% and the conversion of C₆H₅CF(CF₃)₂ was 67%.

## Claims

1. A process for producing an olefinic fluorocarbon selected from the group consisting of (CF₃)₂C=CH₂, CF₃CH=CF₂, CF₂=C(CF₃)OCF₂CHF₂ and C₆H₅C(CF₃)=CF₂, comprising:
contacting the corresponding fluorocarbon starting material selected from the group consisting of (CF₃)₂CFCH₂F, (CF₃)₂CHF, (CF₃)₂CFOCF₂CHF₂ and C₆H₅CF(CF₃)₂, in the vapor phase, with a defluorination reagent selected from the group consisting of carbon, copper, iron, nickel and zinc at an elevated temperature of at least 300°C.

2. The process of Claim 1 wherein (CF₃)₂C=CH₂ is produced from (CF₃)₂CFCH₂F.

## Patentansprüche

1. Verfahren zur Herstellung eines olefinischen Fluorkohlenstoffs, ausgewählt aus der Gruppe, bestehend aus (CF₃)₂C=CH₂, CF₃CH=CF₂, CF₂=C(CF₃)OCF₂CHF₂ und C₆H₅C(CF₃)=CF₂, umfassend:
Inkontaktbringen des entsprechenden Fluorkohlenstoffausgangsmaterials, ausgewählt aus der Gruppe, bestehend aus (CF₃)₂CFCH₂F, (CF₃)₂CHF, (CF₃)₂CFOCF₂CHF₂ und C₆H₅CF(CF₃)₂, in der Dampfphase mit einem Defluorierungsmittel, ausgewählt aus der Gruppe, bestehend aus Kohlenstoff, Kupfer, Eisen, Nickel und Zink, bei einer erhöhten Temperatur von mindestens 300°C.

2. Verfahren nach Anspruch 1, wobei (CF₃)₂C=CH₂ aus (CF₃)₂CFCH₂F hergestellt wird.

## Revendications

1. Procédé de production de fluorocarbones oléfiniques sélectionnés parmi le groupe constitué de (CF₃)₂C=CH₂, CF₃CH=CF₂, CF₂=C(CF₃)OCF₂CHF₂ et C₆H₅C(CF₃) =CF₂, comprenant :
la mise en contact du matériau de départ fluorocarboné correspondant sélectionné parmi le groupe constitué de (CF₃)₂CFCH₂F, (CF₃)₂CHF, (CF₃)₂CFOCF₂CHF₂ et C₆H₅CF(CF₃)₂, en phase vapeur, avec un réactif de défluoration sélectionné parmi le groupe constitué du carbone, du cuivre, du fer, du nickel et du zinc à une température élevée d'au moins 300°C.

2. Procédé selon la revendication 1 dans lequel (CF₃)₂C=CH₂ est produit à partir de (CF₃)₂CFCH₂F.
